(19)

Europäisches
Patentamt
European
Patent Office
Office européen
des brevets

(11) **EP 4 092 679 A1**

(12) **EUROPEAN PATENT APPLICATION**

(43) Date of publication:
**23.11.2022  Bulletin 2022/47**

(21) Application number: **21174919.7**

(22) Date of filing: **20.05.2021**

(51) International Patent Classification (IPC):
**G16B 20/20** (2019.01)        **G16B 40/20** (2019.01)
**G16H 50/20** (2018.01)

(52) Cooperative Patent Classification (CPC):
**G16B 20/20; G16B 40/20; G16H 50/20**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **HULSEN, Tim**
**5656 AE Eindhoven (NL)**
• **VAN DE ZAAG, Pieter**
**5656 AE Eindhoven (NL)**
• **RAAIJMAKERS, Gino**
**5656 AE Eindhoven (NL)**

(74) Representative: **Philips Intellectual Property &
Standards**
**High Tech Campus 52**
**5656 AG Eindhoven (NL)**

(54) **VARIABLE ALLELE FREQUENCY THRESHOLD**

(57)    The present invention relates to monitoring a patient's response to therapy. In order to improve the monitoring of a patient's response to therapy, a method is provided to set a plurality of allele frequency thresholds to accounting for variations among tumours and patients. As the multiple allele frequency thresholds take into account differences between genes, single-nucleotide polymorphisms (SNPs), and/or patients, the multiple allele frequency thresholds may provide significant value to improve personalized therapy selection, disease surveillance, and monitoring to improve patient outcomes.

Fig. 1

EP 4 092 679 A1

**Description**

FIELD OF THE INVENTION

The present invention relates to monitoring a patient's response to a cancer therapy, and in particular to an apparatus and a method for setting an allele frequency threshold, a computer program element, and a computer readable medium.

BACKGROUND OF THE INVENTION

Monitoring therapy response of a cancer patient may be done by analysing patient's samples, such as a liquid biopsy, and following the occurrence of specific mutations over time during treatment. A graph of circulating tumour DNA (ct-DNA) data over time may be very informative when studying the effect of a therapy, particularly when combined with other parameters. In current software, it is possible to manually set an allele frequency threshold to determine whether the allele frequency, at which a certain mutation occurs, is significant.

SUMMARY OF THE INVENTION

There may be a need to improve the monitoring of a patient's response to therapy.

The object of the present invention is solved by the subject-matter of the independent claims, wherein further embodiments are incorporated in the dependent claims. It should be noted that the following described aspects of the invention apply also for the apparatus, the method, the computer program element, and the computer readable medium.

According to a first aspect of the present invention, there is provided an apparatus for determining an allele frequency threshold. The apparatus comprises an input module and a processing module. The input module is configured to receive *allele frequency data* that comprises a plurality of observed variants in a nucleic acid sample of a patient and an allele frequency of each of the plurality of observed variants over time. The processing module configured to set at least two different allele frequency thresholds for the plurality of observed variants, each allele frequency threshold being set for respective one or more observed variants selected from the plurality of observed variants for selecting alleles that have an allele frequency higher than the respective allele frequency threshold. The inventors of the present disclosure have found out that the problem of a single generic allele frequency threshold is that this threshold does not take into account differences between genes and single nucleotide polymorphisms (SNPs). For example, for one specific SNP, a certain allele frequency threshold of 0.4 % might be very high, whereas for another SNP this threshold might be low.

Based on this insight, instead of using a single generic allele frequency threshold, the present disclosure proposes using multiple allele frequency thresholds to account for differences between genes and/or SNPs. Considering the above-mentioned example, two different allele frequency thresholds may be set, e.g. a first allele frequency threshold of 0.3% for one specific SNP and a second allele frequency threshold of 0.5% for another SNP. As the multiple allele frequency thresholds take into account differences between genes and SNPs, the multiple allele frequency thresholds may provide significant value to improve personalized therapy selection (including the use of combination therapies), disease surveillance, and monitoring to improve patient outcomes.

The at least two allele frequency thresholds may be set in various ways.

In some examples, the at least two allele frequency thresholds may be set on a per observed variant basis. For example, if there are five observed variants, five different allele frequency thresholds may be set. Each allele frequency threshold is associated with a respective observed variant.

In some examples, the at least two allele frequency thresholds may not be set on a per observed variant basis. Also taking as an example five observed variants, it may be considered to set two different thresholds. For example, a first allele frequency threshold may be set for three observed variants, whilst a second allele frequency threshold may be set for the remaining two observed variants.

The at least two allele frequency thresholds may be previously calculated based on empirical data from previous studies, and may optionally be optimized using machine learning techniques on a set of data fields from, for example, clinical data, imaging data, and/or digital pathology data.

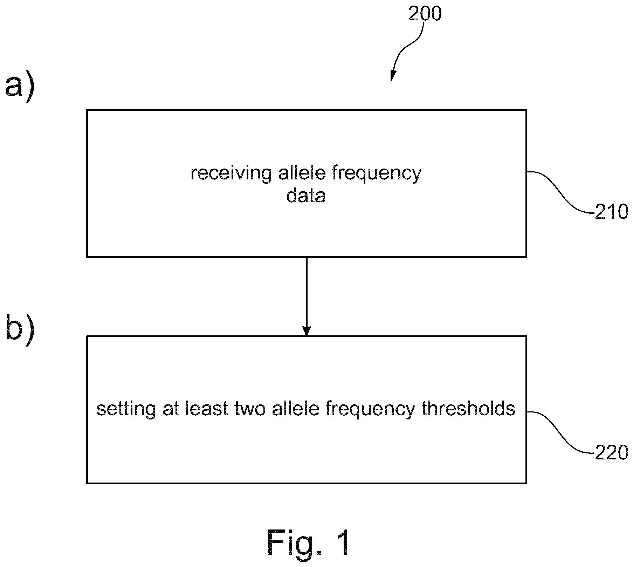

The maximum number of the allele frequency thresholds to be set is equal to the number of observed variants, if the allele frequency thresholds are set on a per observed variant basis. Therefore, if there are five observed variants, the number of the allele frequency thresholds may range between 2 and 5 such as 2, 3, 4 and 5. This will be explained in detail hereinafter and in particular with respect to the example shown in Fig. 1.

The terms "variants", "variations", and "mutations" as used interchangeably herein refer to genetic sequences that are different from a wild type or control sequence.

In some examples, the plurality of observed variants comprise at least two variants from the same gene.

In some examples, the plurality of observed variants comprise at least two variants from different genes.

In some examples, the plurality of observed variants comprise at least two different types of variants, such as SNP mutations and epigenetic mutations.

In some examples, the plurality of observed variants comprise variants of the same type, such SNP mutations.

According to an embodiment of the present invention, the plurality of observed variants comprise at least two different types of mutations selected from a group comprising a SNP mutation, an epigenetic mutation, and a copy number variation (CNV). The processing module is configured to set the at least two different allele frequency thresholds differently between the at least two different mutations.

The inventors of the present disclosure have also found out that another problem of a single generic allele frequency threshold is that this threshold does not take into account differences between different types of mutations, e.g. between SNPs and epigenetic mutations (also referred to as methylation).

Methylation, which is so-called epigenetic change, and CNVs play the role of mutation such as SNPs. The allele frequency is the frequency at which they occur with respect to the wild-type gene. So a patient have an epigenetic changes (i.e. methylation) at some base in the gene (e.g. in the cancer) tissue where the normal tissue does not have this methylation (or vice versa).

In the case of CNVs, the allele frequency can be calculated by comparing the number of duplications, which is higher in tumour tissue than in normal tissue.

Thus, the allele frequency thresholds between different types of mutations may be different.

Based on this insight, the present disclosure proposes setting allele frequency thresholds differently between different types of mutations. For example, if the plurality of observed variants comprises SNP mutations and epigenetic mutations, two different allele frequency thresholds may be set, including one threshold for SNP mutations and one different threshold for epigenetic mutations.

According to an embodiment of the present invention, the processing module is configured to provide the received allele frequency data to a data-driven model to determine the at least two allele frequency thresholds on a per observed variant basis. The data-driven model has been trained based on a training dataset comprising data samples obtained from a plurality of patients to learn a correlation between an allele frequency threshold on a per observed variant basis and or a response to a selection of one or more treatments.

The data-driven model may be a neural network, a support vector machine, a decision tree, or the like.

For training the data-driven model, data samples from previous studies of a plurality of patients are used. The training dataset may comprise pre-treatment data including allele frequencies of given mutations at pre-treatment. The training dataset may also comprise post-treatment data, which may include genomics data indicative of a reduction in tumour DNA, image data indicative of a reduction in tumour size, and/or pathology data indicative of residual mass after resection.

The data-driven model may vary an allele frequency threshold of a given mutation (or given mutations) and select only these patients in the dataset for which the measured allele frequency lies above the threshold. Then the average of the outcome variable may be calculated. The allele frequency threshold, which gives a desirable treatment outcome (e.g. the largest reduction in tumour DNA and/or tumour size), may then be stored.

In this way, multiple data can be brought together into one data model consisting of the most important (derived) data items, which will be used by a machine learning algorithm to determine the optimal allele frequency threshold.

According to an embodiment of the present invention, the processing module is further configured to apply the data-driven model to a group of observed variants to determine a respective allele frequency threshold for each observed variant in the group. The data-driven model has further been trained to learn a correlation between a group of allele frequency thresholds on a per observed variant basis and a desirable treatment outcome given by a selection of one or more treatments.

In other words, the data-driven model may be used for any combination of SNPs (e.g. individual mutations).

In some examples, the data-driven model may be used for any combination of SNPs from the same gene, e.g. KRAS - c.34_35FF>TT - p.G12F, KRAS - c.34_35GG>CT > p.G12L, KRAS - c.35_36GT>TC - p.G12V, and KRAS - c.34G>T -p.G12C.

In some examples, the data-driven model may be used for any combination of SNPs from different genes, e.g. KRAS - c.34_35GG>TT - p.G12F, NRAS - c.34_35GG>CC - p.G12P, BRAF - c.1406G>C - p.G469A, and PIK3CA - c.1624G>A - p.E542K.

The data-driven model may then calculate what optimal allele frequency threshold for each of these SNPs together gives the optimal outcome.

According to an embodiment of the present invention, the training dataset comprises post-treatment data including at least one of genomics data indicative of a reduction in tumour DNA, image data indicative of a reduction in tumour size, and pathology data indicative of residual mass after resection.

The post-treatment data is used for determining a treatment outcome. The outcome may be measured by a reduction in the tumour DNA (e.g. measured by the reduction in the allele frequencies), a reduction in tumour size (as determined from imaging), and/or other parameters (such as residual mass after resection), after treatment.

The genomics data may comprise one or more of SNPs, methylations, and copy number variations (CNVs).

According to an embodiment of the present invention, the input module is further configured to receive clinical data comprising information about the patient. The processing module is further configured to provide the received clinical data to the data-driven model to determine the at least two allele frequency thresholds. The training dataset further comprises information about the plurality of patients for training the data-driven model to determine the at least two allele frequency thresholds on a per patient basis.

The inventors of the present disclosure have also found that a further problem of the generic allele frequency threshold is that this threshold does not take into account differences between patients. For example, for a patient with a very large tumour, the allele frequency threshold might be different than for a patient with a very small tumour.

Based on this insight, the present disclosure further proposes training the data-driven model on a cohort of similar patients in terms of age, gender, cancer type, tumour size, etc. Thus, the trained data-driven model may be used to calculate patient-specific thresholds, which may be beneficial for creating personalized diagnosis and therapy to enable a tailor-made treatment for each patient.

Considering the above-mentioned example, imaging data of the tumour may be used to decide on what threshold to use for that specific patient.

In some examples, the information from the patient may comprise a class of the patient, which may be defined to which cancer-type the patient's disease belongs e.g. a certain type of breast cancer. There are such molecular subgroups in breast cancer such as HER2+ or triple neg. In this way, the allele frequency threshold also takes into account differences between diseases, e.g. between similar mutation (EGFR) in lung and colon cancer and is thus disease-specific.

Other information from the patient, such as age, gender, race, ethnicity, family disease history, weight, body mass index, height, and prior and/or concurrent infections may also be brought into the data-driven model for determining the at least two allele frequency thresholds on a per patient basis.

According to an embodiment of the present invention, the data-driven model comprises a neural network, preferably a Deep Neural Network.

According to an embodiment of the present invention, the processing module is further configured to select, from a plurality of available treatments, one or more treatments that give a desirable treatment outcome based on the at least two allele frequency thresholds.

Thus, it is possible to derive a therapy recommendation from allele frequency data, involving an allele frequency threshold setting. As the multiple allele frequency thresholds take into account differences between genes and SNPs, the multiple allele frequency thresholds may provide significant value to improve therapy selection, disease surveillance, and monitoring.

According to a second aspect of the present invention, there is provided a computer-implemented method for determining an allele frequency threshold. The computer-implemented method comprises:

a) receiving *allele frequency data* that comprises a plurality of observed variants in a nucleic acid sample of a patient and an allele frequency of each of the plurality of observed variants over time; and

b) setting at least two different allele frequency thresholds for the plurality of observed variants, each allele frequency threshold being set for respective one or more observed variants selected from the plurality of observed variants for selecting alleles that have an allele frequency higher than the respective allele frequency threshold.

According to an embodiment of the present invention, the plurality of observed variants comprise at least two different types of mutations selected from a group comprising a SNP mutation, an epigenetic mutation, and a copy number variation (CNV). Step b) further comprises the step of setting the at least two different allele frequency thresholds differently between the at least two different mutations.

According to an embodiment of the present invention, step b) further comprises providing the received allele frequency data to a data-driven model to determine the at least two allele frequency thresholds on a per observed variant basis. The data-driven model has been trained based on a training dataset comprising data samples obtained from a plurality of patients to learn a correlation between an allele frequency threshold on a per observed variant basis and a desirable treatment outcome given by a selection of one or more treatments.

According to an embodiment of the present invention, step b) further comprises applying the data-driven model to a group of observed variants to determine a respective allele frequency threshold for each observed variant in the group. The data-driven model has further been trained to learn a correlation between a group of allele frequency thresholds on a per observed variant basis and a desirable treatment outcome given by a selection of one or more treatments.

According to an embodiment of the present invention, step a) further comprises receiving clinical data comprising information about the patient. Step b) further comprises providing the received clinical data to the data-driven model to determine the at least two allele frequency thresholds. The training dataset further comprises information about the plurality of patients for training the data-driven model to determine the at least two allele frequency thresholds on a per patient basis.

According to a third aspect of the present invention, there is provided a computer program product comprising instructions which, when executed by at least one processing unit, cause the at least one processing unit to perform the steps of the method according to the second aspect and any associated example.

According to a further aspect of the present invention, it is provided a computer readable medium having stored the program element.

As used herein, the data-driven model refers to a trained mathematical model that is parametrized according to a training dataset to reflect a correlation between an allele frequency threshold on a per observed variant basis and a desirable treatment outcome given by a selection of one or more treatments. In some examples, the data-driven model may comprise a data-driven machine learning model. As used herein, the term "machine learning" may refer to a statistical method that enables machines to "learn" tasks from data without explicitly programming. Machine learning techniques may comprise "traditional machine learning" - the workflow in which one manually selects features and then trains the model. Examples of traditional machine learning techniques may include decision trees, support vector machines, and ensemble methods. In some examples, the data-driven model may comprise a data-driven deep learning model. Deep learning is a subset of machine learning modelled loosely on the neural pathways of the human brain. Deep refers to the multiple layers between the input and output layers. In deep learning, the algorithm automatically learns what features are useful. Examples of deep learning techniques may include convolutional neural networks (CNNs) and deep Q networks. A general introduction into machine learning and corresponding software frameworks is described in "Machine Learning and Deep Learning frameworks and libraries for large-scale data mining: a survey"; Artificial Intelligence Review; Giang Nguyen et al., June 2019, Volume 52, Issue 1, pp 77-124.

These and other aspects of the present invention will become apparent from and be elucidated with reference to the embodiments described hereinafter.

BRIEF DESCRIPTION OF THE DRAWINGS

These and other aspects of the invention will be apparent from and elucidated further with reference to the embodiments described by way of examples in the following description and with reference to the accompanying drawings, in which

Fig. 1 shows a flow diagram of a computer-implemented method for setting an allele frequency threshold.
Fig. 2 shows an exemplary graph displaying allele frequencies of four observed variants and MR parameters on five different time points.
Fig. 3 shows an apparatus for setting an allele frequency threshold.
Fig. 4 shows a system for monitoring a patient's response to a cancer therapy.

It should be noted that the figures are purely diagrammatic and not drawn to scale. In the figures, elements which correspond to elements already described may have the same reference numerals. Examples, embodiments or optional features, whether indicated as non-limiting or not, are not to be understood as limiting the invention as claimed.

DETAILED DESCRIPTION OF EMBODIMENTS

Fig. 1 illustrates a flow diagram of a computer-implemented method 200 for setting an allele frequency threshold.

In step 210, i.e. step a), allele frequency data is received. The allele frequency data comprises a plurality of observed variants in a nucleic acid sample of a patient and an allele frequency of each of the plurality of observed variants over time.

The allele frequency data may have been obtained based on an analysis of a biomarker in a patient sample and stored in a dataset. The patient sample may comprise, e.g. a tissue sample, a body fluid, a cell sample, or a stool sample. In some examples, the patient sample may comprise a body fluid, such as whole blood, saliva, tears, sweat, sputum, or urine. In some examples, only a portion of the whole blood, such as blood plasma which contains the cell free nucleic acid is used. In other examples, the patient sample is a tissue sample, such as a formalin-fixed parafin-embedded (FFPE) tissue sample, a fresh frozen (FF) tissue sample, or a combination thereof.

For example, tumours may release multiple biomarkers in the bloodstream, such as ct-DNA, circulating tumour cells (CTCs), exosomes, and platelets. ct-DNA originates from dying tumour cells. CTCs are shed into the bloodstream from primary tumours and metastases. Exosomes are cell-derived vesicles containing tumour messenger RNA (mRNA), microRNA (miRNA), protein, and doublestranded DNA (dsDNA). Platelets are reported to pick up tumour RNA in circulation. Currently, ct-DNA and CTCs may be clinically the most relevant biomarkers used to monitor therapy response. Of these, ct-DNA may be commonly used, as CTCs may occur at low count (e.g., less than 10) in most cancer patients unless they have metastatic disease.

Since different cancer patients may carry different mutations in their tumours, one may need to observe a plurality of variants, i.e., mutations and/or epigenetic changes, in order to assure that at least some of them can be found in the biomarker, such as ct-DNA. For example, breast cancer is a very heterogeneous disease with a wide range of genetic changes that may occur, yet all with a limited frequency among the patient population, e.g., at 15% or less. For example, an analysis of the various genetic changes relevant to breast cancer and their typical occurrence in patients is illustrated in Table 1 compiled at Philips Research:

| Gene | #studies | frequency |
|---|---|---|
| PIK3CA | 9 | 39% |
| TP53 | 9 | 35% |
| MAP3K1 | 8 | 12% |
| GATA3 | 8 | 11% |
| CDH1 | 7 | 9.2% |
| KMT2C/MLL3 | 9 | 7.8% |
| PTEN | 6 | 6.8% |
| ARID1B | 2 | 6.5% |
| CDKN2A | 3 | 5.3% |
| SMAD4 | 2 | 5.3% |

Table 1

Table 1 shows the frequency that certain genes (left column) are mutated (right column) in a general population of women having breast cancer. The middle column gives how many studies in the scientific literature have reported on this mutation from which the frequency was derived.

Each of the plurality of observed variants is longitudinally tracked through time at a plurality of time points. In some example, the plurality of time points may comprise a first time point (or more time points) before a treatment and a second time point (or more time points) after the treatment. For instance, the treatment could be cytostatic drugs, a targeted drug therapy or radiation therapy or any combination of these.

In step 220, i.e. step b), at least two different allele frequency thresholds are set for the plurality of observed variants. Each allele frequency threshold is set for respective one or more observed variants selected from the plurality of observed variants for selecting alleles that have an allele frequency higher than the respective allele frequency threshold.

To put it in another way, instead of using a single generic allele frequency threshold, multiple allele frequency thresholds are introduced to account for differences between e.g. genes and/or SNPs. The multiple allele frequency thresholds are set differently between different mutations in a single patient.

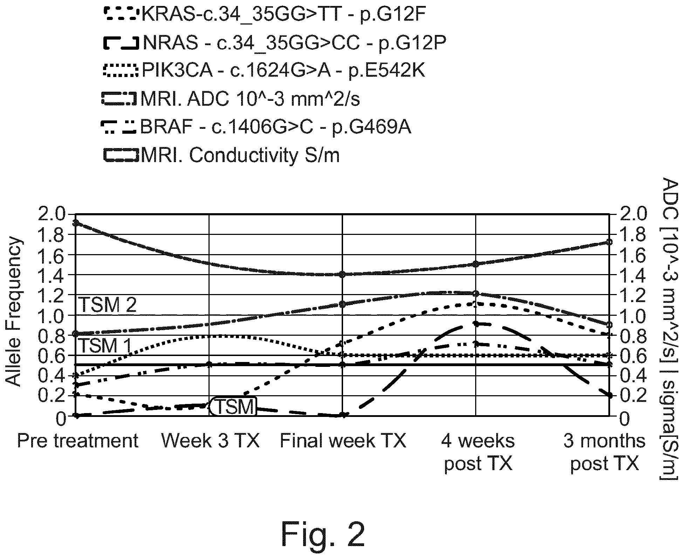

Fig. 2 shows an exemplary graph displaying allele frequencies of four observed variants and MR parameters on five different time points. The horizontal TSM (Therapy Selection Module) line is an allele frequency threshold that can be used to select specific alleles that have a frequency higher than that threshold.

In the example of Fig. 2, two different thresholds are set, namely TSM1 and TSM2. In an example, the allele frequency threshold TSM1 may be set for two observed variants (e.g. KRAS - c.34_35GG>TT - p.G12F and NRAS - c.34_35GG>CC - p.G12P), whilst the allele frequency threshold TSM2 may be set for the remaining two observed variants (e.g. BRAF - c. 1406G>C - p.G469A, and PIK3CA - c.1624G>A - p.E542K). In another example, the allele frequency threshold TSM1 may be set for three observed variants (e.g. KRAS - c.34_35GG>TT - p.G12F, NRAS - c.34_35GG>CC - p.G12P, and BRAF - c. 1406G>C - p.G469A), whilst a second allele frequency threshold may be set for the remaining one observed variant (e.g. PIK3CA - c. 1624G>A - p.E542K).

Also taking as an example four observed variants, it may be considered to set three different thresholds. For example, a first allele frequency threshold may be set for one observed variant; a second allele frequency threshold may be set for two observed variants; and a third allele frequency threshold may be set for the remaining one observed variants.

As a further example, it may be considered to set an allele frequency threshold on a per observed variant basis.

For example, in the example of Fig. 2, four different allele frequency thresholds may be set.

The at least two allele frequency thresholds may be displayed when a user input is received that selects 1) a certain patient and 2) a specific mutation (or several specific mutations). In the example of Fig. 1, the two allele frequency thresholds TSM1 and TSM 2 are both displayed. In some examples (not shown), the user may select one allele frequency threshold to display.

The at least two different allele frequency thresholds may be previously calculated e.g. using empirical approaches. For example, empirical bottom-up analysis using allele frequency thresholds of e.g. 0.001%, 0.005%, 0.01%, 0.05%, and 0.1% may be adopted from a previous study.

For example, it is possible to use a variable allele frequency threshold for pre-treatment data, which was previously calculated from another dataset, as the allele frequency threshold.

In order to find the threshold that gives improved results, machine learning techniques may be used on a wide range of data items derived from clinical data, genomics data, imaging data and/or digital pathology data.

For example, step b) may further comprise the step of providing the received allele frequency data to a data-driven model to determine the at least two allele frequency thresholds on a per observed variant basis. The data-driven model has been trained based on a training dataset comprising data samples obtained from a plurality of patients to learn a correlation between an allele frequency threshold on a per observed variant basis and a desirable treatment outcome given by a selection of one or more treatments.

Taking Kirsten Rat Sarcoma (KRAS) mutation data as an example, for each SNP in the KRAS gene and for each patient from a large dataset (e.g. 1000 patients), it may use as feature the allele frequency at pre-treatment, such as:

KRAS - c.34_35FF>TT - p.G12F allele frequency at pre-treatment;
KRAS - c.34_35GG>CT > p.G12L allele frequency at pre-treatment;
KRAS - c.35_36GT>TC - p.G12V allele frequency at pre-treatment;
KRAS - c.34G>T - p.G12C allele frequency at pre-treatment;
KRAS - c.34G>C - p.G12R allele frequency at pre-treatment.

The response to a selection of one or more treatments may be measured based on post-treatment data. In some examples, outcome may be measured using genomics data obtained from liquid biopsies, such as ct-DNA data, indicative of a reduction in tumour DNA. As alternative or additional to SNPs, other genomics data could be used, such as methylations or copy number variations (CNVs). In some examples, outcome may be measured using image data indicative of a reduction in tumour size. The reduction in tumour size could be determined by any imaging type, such as magnetic resonance imaging (MRI), ultrasound imaging (US), positron emission tomography (PET), and/or computed tomography (CT). The image data may be obtained from the acquisition of e.g. magnetic resonance signals, or separately such as offline or in the Cloud. In some examples, outcome may be measured using pathology data indicative of residual mass after resection.

To determine the allele frequency thresholds, the machine learning algorithm may vary an allele frequency threshold of a given mutation (or given mutations) and select only these patients in the dataset for which the measured allele frequency lies above the threshold. Then the average of the outcome variable may be calculated. The allele frequency threshold which gives a desirable treatment outcome (e.g. the largest reduction in tumour DNA and/or tumour size) may then then stored. In this way, it is determined below which thresholds of allele frequencies of given mutations have to drop to consider that the patient is responding to a treatment irrespective of the nature of the treatment.

The machine learning algorithm may be used for any combination of SNPs (e.g. individual mutations).

In the above example, the machine learning algorithm is used for a combination of SNPs from the same gene.

In some examples, the machine learning algorithm may be used for a combination of SNPs from different genes. For example, the following SNPs shown in Fig. 1 may be selected:

$$KRAS - c.34\_35GG{>}TT - p.G12F;$$

$$NRAS - c.34\_35GG{>}CC - p.G12P;$$

$$BRAF - c.1406G{>}C - p.G469A;$$

and PIK3CA – c.1624G>A – p.E542K.

Optionally, step b) may further comprise applying the data-driven model to a group of observed variants (e.g. two combinations of SNPs described above) to determine a respective allele frequency threshold for each observed variant in the group. The data-driven model has further been trained to learn a correlation between a group of allele frequency thresholds on a per observed variant basis and a desirable treatment outcome given by a selection of one or more treatments. In other words, the machine learning algorithm may calculate what optimal allele frequency threshold for each of these observed variants in the group together gives the optimal outcome.

For example, the machine learning algorithm may also use the above-described approach, but vary values of the multiple allele frequency thresholds in the group and determine a combination of allele frequency threshold values that gives a desirable treatment outcome.

Optionally, multiple allele frequency thresholds may be introduced to account for differences between different types of mutations. For example, the plurality of observed variants comprise at least two different types of mutations selected from a group comprising an SNP mutation, an epigenetic mutation, and a CNV. Step b) further comprises the step of setting the at least two different allele frequency thresholds differently between the at least two different mutations. The allele frequency thresholds for different types of mutations may be previously calculated based on empirical data from previous studies and may optionally be optimized using machine learning techniques as described above.

Optionally, step a) may further comprise the step of receiving clinical data comprising information about the patient. In some examples, the clinical data may include imaging data of tumours. In some examples, the information from the patient may comprise a class of the patient, which may be defined to which the patient belongs e.g. a certain type of breast cancer. In some examples, information from the patient, such as age, gender, race, ethnicity, family disease history, weight, body mass index, height, and prior and/or concurrent infections may also be brought into the data-driven model. Step b) may further comprise the step of providing the received clinical data to the data-driven model to determine the at least two allele frequency thresholds. The training dataset may further comprise information about the plurality of patients for training the data-driven model to determine the at least two allele frequency thresholds on a per patient basis.

In this way, the data-driven model may be trained on a cohort of similar patients in terms of age, gender, cancer type, etc. Thus, the allele frequency thresholds may be set differently for patients with different tumour sizes, disease types, genders, ages, etc. A patient-specific threshold may be preferred for software related to Precision Medicine, as this field is all about creating personalized diagnosis and therapy to allow a tailor-made treatment for each patient.

Optionally, the computer-implemented method 100 may further comprise the step of selecting, from a plurality of available treatments, one or more treatments that give a desirable treatment outcome based on the at least two allele frequency thresholds. Thus, setting multiple allele frequency thresholds to account for differences between genes, SNPs, and/or patients can inform personalized treatment selection, including the use of combination therapies, to improve patient outcomes.

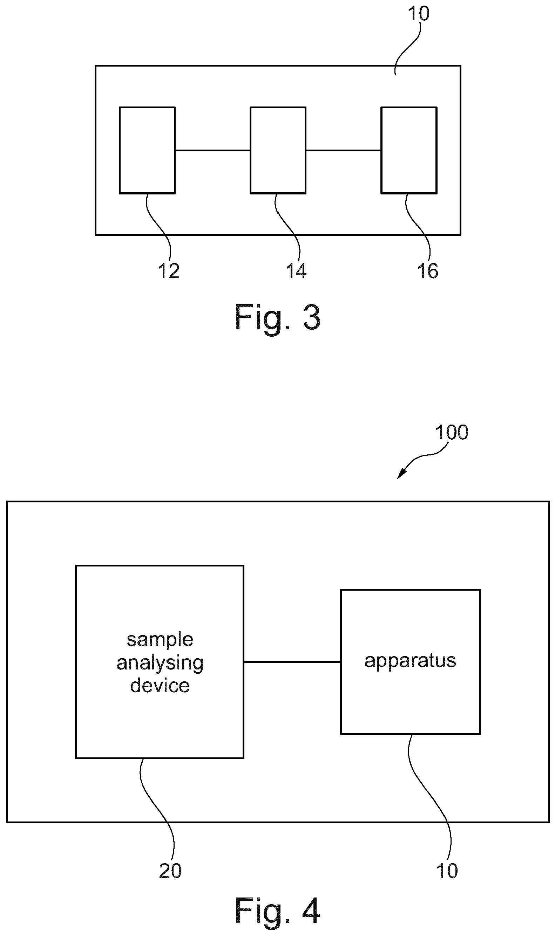

Fig. 3 schematically shows an apparatus 10 for setting an allele frequency threshold. The apparatus 10 comprises an input module 12 and a processing module 14. Each module may be part of, or include an Application Specific Integrated Circuit (ASIC), an electronic circuit, a processor (shared, dedicated, or group) and/or memory (shared, dedicated, or group) that execute one or more software or firmware programs, a combinational logical circuit, and/or other suitable components that provide the described functionality.

The input module 12 is configured to receive *allele frequency data* that comprises a plurality of observed variants in a nucleic acid sample of a patient and an allele frequency of each of the plurality of observed variants over time.

The processing module 14 is configured to set at least two different allele frequency thresholds for the plurality of observed variants, each allele frequency threshold being set for respective one or more observed variants selected from the plurality of observed variants for selecting alleles that have an allele frequency higher than the respective allele frequency threshold. The number of the allele frequency thresholds may range between 2 and the number of the observed variants. For example, if there are nine observed variants, the number of the allele frequency thresholds may range between 2 and 9, such as 2, 3, 4, 5, 6, 7, 8 and 9.

Optionally, as illustrated in Fig. 3, the apparatus 10 may further comprise an output unit 16 for outputting the results. In an example, the output unit 16 may comprise a display.

The apparatus 10 may be configured to be carry out any one of the above-described method steps.

In some examples, the plurality of observed variants may comprise at least two different types of mutations selected from a group comprising a SNP mutation, an epigenetic mutation, and a CNV. The processing module may be configured to set the at least two different allele frequency thresholds differently between the at least two different mutations

In some examples, the allele frequency thresholds may be determined using machine learning approaches. For example, the processing module 14 may be configured to provide the received allele frequency data to a data-driven model to determine the at least two allele frequency thresholds on a per observed variant basis. The data-driven model has been trained based on a training dataset comprising data samples obtained from a plurality of patients to learn a correlation between an allele frequency threshold on a per observed variant basis and or a response to a selection of one or more treatments. The training dataset may comprise pre-treatment data including allele frequencies of given mutations at pre-treatment. The training dataset may comprise post-treatment data including genomics data indicative of a reduction in tumour DNA, image data indicative of a reduction in tumour size, and/or pathology data indicative of residual mass after resection.

In some examples, the processing module 14 may be further configured to apply the data-driven model to a group of observed variants to determine a respective allele frequency threshold for each observed variant in the group. The data-driven model has further been trained to learn a correlation between a group of allele frequency thresholds on a per observed variant basis and a desirable treatment outcome given by a selection of one or more treatments.

In some examples, the input module 12 may be further configured to receive clinical data comprising information about the patient. The processing module 14 may be further configured to provide the received clinical data to the data-driven model to determine the at least two allele frequency thresholds. The training dataset may further comprise information about the plurality of patients for training the data-driven model to determine the at least two allele frequency thresholds on a per patient basis.

Fig. 4 schematically shows a system 100 for monitoring a patient's response to a cancer therapy. The system 100 comprises an apparatus 10 as described above and a sample analysing device 20.

The apparatus 10 may be any computing device, including desktop and laptop computers, smartphones, tablets, etc. The apparatus 10 may be a general-purpose device or a device with a dedicated unit of equipment suitable for providing the below-described functionality. In the example of Fig. 4, the components of the apparatus 10 are shown as integrated in one single unit. However, in alternative examples, some or all components may be arranged as separate modules in a distributed architecture and connected in a suitable communication network. The apparatus 10 and its components may be arranged as dedicated field-programmable gate arrays (FPGAs) or as hardwired standalone chips. In some examples, the apparatus 10 or some of its components may be resident in sample analysing device 20 running as software routines.

The sample analysing device 20 is configured for analysing a patient sample to obtain allele frequency data of the patient to be output to the apparatus 10. In an example, the sample analysing device 20 may be sequencers, such as the HiSeq of Illumina or the IonTorrent of ThermoFisher. In another example, the sample analysing device 20 may be mass-spectrometry systems, such as the Agena Biosciences MassARRAY system. In a further example, the sample analysing device 20 may be a digital droplet polymerase chain reaction (PCR) system, such as the Bio-Rad QX200 digital droplet PCR. In a further example, the sample analysing device 20 may be a multiplex PCR system, such as the Biocartis Idylla platform.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1.  An apparatus (10) for setting an allele frequency threshold, comprising:

    - an input module (12) configured to receive *allele frequency data* that comprises a plurality of observed variants in a nucleic acid sample of a patient and an allele frequency of each of the plurality of observed variants over time; and
    - a processing module (14) configured to set at least two different allele frequency thresholds for the plurality of observed variants, each allele frequency threshold being set for respective one or more observed variants selected from the plurality of observed variants for selecting alleles that have an allele frequency higher than the respective allele frequency threshold.

2.  Apparatus according to claim 1,
    wherein the plurality of observed variants comprises at least two different types of mutations selected from a group comprising a single-nucleotide polymorphism, SNP, mutation, an epigenetic mutation, and a copy number variation, CNV; and
    wherein the processing module is configured to set the at least two different allele frequency thresholds differently between the at least two different mutations.

3.  Apparatus according to claim 1 or 2,
    wherein the processing module is configured to provide the received allele frequency data to a data-driven model to determine the at least two allele frequency thresholds on a per observed variant basis; and
    wherein the data-driven model has been trained based on a training dataset comprising data samples obtained from a plurality of patients to learn a correlation between an allele frequency threshold on a per observed variant basis and or a response to a selection of one or more treatments.

4.  Apparatus according to claim 3,
    wherein the processing module is further configured to apply the data-driven model to a group of observed variants to determine a respective allele frequency threshold for each observed variant in the group; and
    wherein the data-driven model has further been trained to learn a correlation between a group of allele frequency thresholds on a per observed variant basis and a desirable treatment outcome given by a selection of one or more treatments.

5.  Apparatus according to claim 3 or 4,
    wherein the training dataset comprises post-treatment data including at least one of:

    - genomics data indicative of a reduction in tumour DNA;
    - image data indicative of a reduction in tumour size; and
    - pathology data indicative of residual mass after resection.

6. Apparatus according to any one of claims 3 to 5,
wherein the input module is further configured to receive clinical data comprising information about the patient;
wherein processing module is further configured to provide the received clinical data to the data-driven model to determine the at least two allele frequency thresholds; and
wherein the training dataset further comprises information about the plurality of patients for training the data-driven model to determine the at least two allele frequency thresholds on a per patient basis.

7. Computer-implemented method according to any one of claims 3 to 6,
wherein the data-driven model comprises a neural network, preferably a Deep Neural Network.

8. Apparatus according to any one of the preceding claims,
wherein the processing module is further configured to select, from a plurality of available treatments, one or more treatments that give a desirable treatment outcome based on the at least two allele frequency thresholds.

9. A computer-implemented method (200) for setting an allele frequency threshold, comprising:

a) receiving (210) *allele frequency data* that comprises a plurality of observed variants in a nucleic acid sample of a patient and an allele frequency of each of the plurality of observed variants over time; and
b) setting (220) at least two different allele frequency thresholds for the plurality of observed variants, each allele frequency threshold being set for respective one or more observed variants selected from the plurality of observed variants for selecting alleles that have an allele frequency higher than the respective allele frequency threshold.

10. Computer-implemented method according to claim 9,
wherein the plurality of observed variants comprises at least two different types of mutations selected from a group comprising a single-nucleotide polymorphism, SNP, mutation, an epigenetic mutation, and a copy number variation, CNV; and
wherein step b) further comprises the step of setting the at least two different allele frequency thresholds differently between the at least two different mutations.

11. Computer-implemented method according to claim 9 or 10,
wherein step b) further comprises the step of providing the received allele frequency data to a data-driven model to determine the at least two allele frequency thresholds on a per observed variant basis; and
wherein the data-driven model has been trained based on a training dataset comprising data samples obtained from a plurality of patients to learn a correlation between an allele frequency threshold on a per observed variant basis and a desirable treatment outcome given by a selection of one or more treatments.

12. Computer-implemented method according to claim 11,
where step b) further comprises applying the data-driven model to a group of observed variants to determine a respective allele frequency threshold for each observed variant in the group; and
wherein the data-driven model has further been trained to learn a correlation between a group of allele frequency thresholds on a per observed variant basis and a desirable treatment outcome given by a selection of one or more treatments.

13. Computer-implemented method according to claim 11 or 12,
wherein step a) further comprises receiving clinical data comprising information about the patient;
wherein step b) further comprises providing the received clinical data to the data-driven model to determine the at least two allele frequency thresholds; and
wherein the training dataset further comprises information about the plurality of patients for training the data-driven model to determine the at least two allele frequency thresholds on a per patient basis.

14. A computer program product comprising instructions which, when executed by at least one processing unit, cause the at least one processing unit to perform the steps of the method according to any one of claims 9 to 13.

15. Computer readable medium having stored the program product of claim 14.

200

a)

receiving allele frequency
data

210

b)

setting at least two allele frequency thresholds

220

Fig. 1

KRAS-c.34_35GG>TT - p.G12F
NRAS - c.34_35GG>CC - p.G12P
PIK3CA - c.1624G>A - p.E542K
MRI. ADC 10^-3 mm^2/s
BRAF - c.1406G>C - p.G469A
MRI. Conductivity S/m

Fig. 2

10

Fig. 3

100

sample
analysing
device

apparatus

20

10

Fig. 4

Europäisches
Patentamt

European
Patent Office

Office européen
des brevets

# EUROPEAN SEARCH REPORT

Application Number

EP 21 17 4919

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ALKES L. PRICE ET AL: "Pooled Association Tests for Rare Variants in Exon-Resequencing Studies", THE AMERICAN JOURNAL OF HUMAN GENETICS, vol. 86, no. 6, 11 June 2010 (2010-06-11), pages 832-838, XP055464451, US ISSN: 0002-9297, DOI: 10.1016/j.ajhg.2010.04.005 | 1-4, 7-12,14, 15 | INV. G16B20/20 G16B40/20 G16H50/20 |
| A | * the whole document * * in particular * * page 833 - page 836 * | 5,6,13 | |
| X | SHEARER A. ELIOT ET AL: "Utilizing Ethnic-Specific Differences in Minor Allele Frequency to Recategorize Reported Pathogenic Deafness Variants", THE AMERICAN JOURNAL OF HUMAN GENETICS, vol. 95, no. 4, 2 October 2014 (2014-10-02), pages 445-453, XP055855985, US ISSN: 0002-9297, DOI: 10.1016/j.ajhg.2014.09.001 | 1-4, 7-12,14, 15 | |
| A | * the whole document * | 5,6,13 | TECHNICAL FIELDS SEARCHED (IPC)  G16B G16H |
| X | WO 2020/051529 A1 (SEQUENOM INC [US]) 12 March 2020 (2020-03-12) | 1,9,14, 15 | |
| A | * page 44 * | 2-8, 10-13 | |

The present search report has been drawn up for all claims

| Place of search | Date of completion of the search | Examiner |
|---|---|---|
| Munich | 28 October 2021 | Rákossy, Z |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 17 4919

This annex lists the patent family members relating to the patent documents cited in the above-mentioned European search report.
The members are as contained in the European Patent Office EDP file on
The European Patent Office is in no way liable for these particulars which are merely given for the purpose of information.

28-10-2021

| Patent document cited in search report | Publication date | Patent family member(s) | | Publication date |
|---|---|---|---|---|
| WO 2020051529 A1 | 12-03-2020 | CA | 3107467 A1 | 12-03-2020 |
| | | EP | 3847280 A1 | 14-07-2021 |
| | | US | 2021301342 A1 | 30-09-2021 |
| | | WO | 2020051529 A1 | 12-03-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GIANG NGUYEN et al.** Machine Learning and Deep Learning frameworks and libraries for large-scale data mining: a survey. *Artificial Intelligence Review,* June 2019, vol. 52 (1), 77-124